# EUROPEAN PATENT SPECIFICATION

(11) **EP 2 666 416 B1**
(45) Date of publication and mention of the grant of the patent: **16.03.2016**
(21) Application number: 13168805.3
(22) Date of filing: 22.05.2013
(51) Int. Cl.: A61B 17/00, A61B 17/02, A61B 17/34

(54) **Removable medical retractor tip**
Abnehmbare medizinische Retraktorspitze
Pointe de rétracteur médical amovible

(30) Priority: 25.05.2012 US 201261651803 P; 04.03.2013 US 201313783570
(43) Date of publication of application: 27.11.2013
(73) Proprietor: Cook Medical Technologies LLC, Bloomington, IN 47404 (US)
(72) Inventor: Stevenson, Andrew R.L., Newport, Queensland 4021 (AU); Burleigh, Tiffany, Logan Central, Queensland 4114 (AU); Ford, Michael William, Palm Beach, Queensland 4221 (AU); Gutierrez, Carolina, Moorooka, Queensland 4105 (AU); Junger, Michael Carl, Brookfield, Queensland 4069 (AU); Macnaughtan, James, Fig Tree Pocket, Queensland 4069 (AU); Vizcay-Wilson, Caitlin, Bonogin, Queensland 4213 (AU)
(74) Representative: Garratt, Peter Douglas

(56) References cited:
- EP-A1- 0 664 992
- WO-A1-2006/088578
- WO-A2-2004/037070
- GB-A- 2 481 056
- US-A- 5 372 588
- US-A- 5 817 061
- US-A1- 2012 065 589
- US-B1- 7 485 092

## Description

### TECHNICAL FIELD

The present invention relates to medical devices and more specifically, instruments used in conjunction with laparoscopic surgery.

### BACKGROUND

Laparoscopic surgical procedures generally involve inflating a bodily cavity with a gas, such as the abdomen, to provide better visibility of the surgical site. Such gasses may include carbon dioxide. After the surgical site is insufflated, the bodily cavity may be punctured using a trocar device for the purposes of inserting surgical tools such as a laparoscopic camera, cutting and manipulating tools, etc. For example, when performing laparoscopic surgery in the abdominal area, the trocar device is utilized to puncture the peritoneum. Thereafter, a laparoscopic retractor may be inserted through the puncture site and directed to the targeted anatomy to assist in retracting and holding certain bodily organs and tissue, thereby exposing the surgical field for the procedure. Maintaining adequate vision of the target or surgical field during laparoscopic procedures is critical to a successful laparoscopic procedure, as inadequate ability to visualize anatomical structures is a common complication of laparoscopic procedures, and can lead to conversion from a laparoscopic to an open procedure.

Laparoscopic surgery has many advantages over traditional open surgery in that it generally takes less time to complete, the patient is likely to experience less severe post-operative pain, and the incisions leave less noticeable scarring as compared to open surgery. Additionally, hospital recovery time and costs are generally reduced.

Despite the benefits of laparoscopic surgery, such surgery is often difficult to perform due to the effort required to arrive at adequate exposure of the surgical field. This is especially true in the case of inserting a retractor into a larger patient due to, for example, the distribution of adipose tissue over the preferred insertion site. For example, after the initial insertion is made, the retractor must be navigated through numerous layers of materials, including the skin, adipose tissue, etc. to reach the abdominal cavity and from there the target anatomy site. The materials through which the retractor is navigated are malleable and flexible, and accordingly, the incision does not remain fixed relative to the skin, adipose tissue, etc. Thus, inserting the retractor through the layers of material, especially when the patient has a thicker layer of adipose tissue over the insertion site, is difficult and results in a phenomenon called "tenting", wherein the retractor becomes stuck in the layers of material that have moved relative to the insertion point. This delays the procedure until the end of the retractor finally locates the incision into the abdominal cavity. Because laparoscopic retractors typically have a rounded end to facilitate easy insertion though the skin and into the abdominal cavity, the length of the end dictates how much adipose tissue can be accommodated. The more the adipose layers must be compressed to insert the insertion end, the more likely the incision path becomes difficult to navigate. Although the insertion end of the retractor could be lengthened, a long retractor end impedes the medical procedure once it has been inserted, particularly in surgical areas having space constraints such as the pelvic area. Accordingly, present retractors do not provide an adequate solution to performing efficient laparoscopic surgery on those patients with additional adipose tissue over the preferred insertion site.

Reference is directed to US 7,485,092 which discloses an apparatus and method for harvesting vessels in the body of a patient including manual manipulation of a rigid dissecting endoscope and the reconfiguration thereof to facilitate tissue dissection and tissue dilation. A tissue dilating collar may optionally be placed at the distal end of the dissecting endoscope. Reference is further directed to US 2012/0065589 which discloses a cannula assembly having a plurality of outwardly-biased flexible fins capable of inward movement such that the fins converge upon insertion of a trocar device. The fins include a slot formed therein, with a corresponding raised feature on the trocar shaft capable of engaging the slots. Inward movement of the trocar within the cannular lumen causes the fins to converge. An additional embodiment utilizes outwardly-biased flexible fins that lock together in a closed position, with corrugated features in the fin inner surface that contact the trocar shaft such that, upon insertion of the trocar, the fins unlock and splay outward.

Document US5817061 discloses the subject-matter of the preamble of claim 1. The invention is defined in claim 1. Preferred embodiments are recited in the dependent claims.

### BRIEF SUMMARY

In a first aspect, a removable tip is provided comprising a substantially conical-shaped body comprising a proximal body portion and a distal body portion, wherein the proximal body portion comprises a substantially hollow portion and is configured for receiving a distal portion of a medical retractor, and wherein the distal body portion is atraumatic.

In some embodiments, when said proximal body portion is engaged with said distal portion of a medical retractor, said distal body portion may be movable relative to said proximal body portion and to said distal portion of said medical retractor, such that a passageway of sufficient size to accommodate said distal portion of said medical retractor is opened in said conical-shaped body and such that said removable tip may thereafter slide along the length of the medical retractor, with the medical retractor being accommodated in said passageway. Suitably, the removable tip comprises a disengagement means that is actuable to cause said moment of said distal body portion relative to said proximal body portion and to said distal portion of said medical retractor. The opening of a passageway of sufficient size to accommodate said distal portion of said medical retractor may include the enlargement of an existing passageway to a sufficient size to accommodate said distal portion of said medical retractor, or may include a snap point breaking a portion of a supporting neck disposed between the proximal body portion and the distal body portion. Suitably, said passageway may be opened by the tearing of a region of weakness in said conical-shaped body. In some embodiments, said passageway may be opened by the tearing of two regions of weakness, or a plurality of regions of weakness. Suitably, the or each region of weakness may comprise a plurality of perforations disposed on the substantially conical-shaped body. Such perforations may be disposed longitudinally along, circumferentially around, or spirally about the conical-shaped body. Alternatively, or in addition, the or each region of weakness may comprise a break line, which may suitably be disposed longitudinally. In some embodiments, the removable tip may comprise disengagement means actuable to transform said conical-shaped body into a sliding configuration, wherein said conical-shaped body may slide along the length of said medical retractor. In other embodiments, the removable tip may comprise disengagement means comprising one or more regions of weakness (including any of the features above) that may be torn so as to allow said removable tip to disengage from said distal portion of said medical retractor. Suitably, a pull cord may be attached to the distal or proximal end of said conical-shaped body so as to enable the tearing of said region(s) of weakness. More generally, a pull cord may be attached to the distal or proximal end of said conical-shaped body so as to enable the removable tip to disengage from said distal portion of said medical retractor.

In a second aspect, a medical retractor system is provided, comprising a removable tip having a substantially conical-shaped body comprising a proximal body portion and a distal body portion, wherein the proximal body portion comprises a substantially hollow portion and is configured for receiving a distal portion of a medical retractor, and wherein the distal body portion is atraumatic; a disengagement means in communication with the substantially conical-shaped body configured to disengage the substantially conical-shaped body from a distal portion of a medical retractor; and a medical retractor having a proximal portion and a distal portion, wherein the distal portion is connected to the removable tip.

In some embodiments, the disengagement means may be actuable to move said distal body portion relative to said proximal body portion and to said distal portion of said medical retractor, such that a passageway of sufficient size to accommodate said distal portion of said medical retractor is opened in said conical-shaped body and such that said removable tip may thereafter slide along the length of the medical retractor, preferably wherein said passageway is opened by the tearing of a region of weakness in said conical-shaped body. The opening of a passageway of sufficient size to accommodate said distal portion of said medical retractor may include the enlargement of an existing passageway to a sufficient size to accommodate said distal portion of said medical retractor, or may include a snap point breaking a portion of a supporting neck disposed between the proximal body portion and the distal body portion. Suitably, said passageway may be opened by the tearing of a region of weakness in said conical-shaped body. In some embodiments, said passageway may be opened by the tearing of two regions of weakness, or a plurality of regions of weakness. Suitably, the or each region of weakness may comprise a plurality of perforations disposed on the substantially conical-shaped body. Such perforations may be disposed longitudinally along, circumferentially around, or spirally about the conical-shaped body. Alternatively, or in addition, the or each region of weakness may comprise a break line, which may suitably be disposed longitudinally. Suitably, the medical retractor system may comprise a plurality of removable tips of different sizes or, more specifically, of different overall lengths. In some embodiments, the removable tip may comprise disengagement means actuable to transform said conical-shaped body into a sliding configuration, wherein said conical-shaped body may slide along the length of said medical retractor. In other embodiments, the removable tip may comprise disengagement means comprising one or more regions of weakness (including any of the features above) that may be torn so as to allow said removable tip to disengage from said distal portion of said medical retractor. Suitably, a pull cord may be attached to the distal or proximal end of said conical-shaped body so as to enable the tearing of said region(s) of weakness. More generally, a pull cord may be attached to the distal or proximal end of the conical-shaped body so as to enable the removable tip to disengage from said distal portion of said medical retractor.

In a third aspect, a method for using a medical retractor system is provided, comprising the steps of providing a medical retractor comprising a proximal portion and a distal portion; and providing a removable tip comprising a substantially conical-shaped body comprising a proximal body portion and a distal body portion, wherein the proximal body portion comprises a substantially hollow portion and is configured for receiving the distal portion of the medical retractor, and wherein the distal body portion is atraumatic.

In some aspects a plurality of removable tips of different sizes may be provided, the method suitably further comprising selecting an appropriately-sized one of said removable tips based on the clinical facts and circumstances. The plurality of removable tips of different sizes may more specifically be of different overall lengths.

### BRIEF DESCRIPTION OF THE SEVERAL VIEWS OF THE DRAWINGS

The embodiments will be further described in connection with the attached drawing figures. It is intended that the drawings included as a part of this specification be illustrative of the exemplary embodiments and should in no way be considered as a limitation on the scope of the invention. Indeed, the present disclosure specifically contemplates other embodiments not illustrated but intended to be included in the claims. Moreover, it is understood that the figures are not necessarily drawn to scale.
Fig. 1A illustrates a perspective view of an exemplary retractor attached to an exemplary removable tip;
Fig. 1B illustrates a perspective view of the exemplary retractor detached from the exemplary removable tip illustrated in Fig. 1A;
Fig. 1C illustrates a perspective view of a portion of the exemplary retractor detached from the exemplary removable tip illustrated in Fig. 1B at the circle 1C;
Fig. 2A illustrates a perspective view of an exemplary retractor attached to another exemplary removable tip;
Fig. 2B illustrates a bottom perspective view of the exemplary removable tip illustrated in Fig. 2A;
Fig. 2C illustrates a perspective view of the exemplary removable tip illustrated in Fig. 2A just prior to disengagement from the exemplary retractor;
Fig. 2D illustrates a perspective view of the exemplary removable tip illustrated in Fig. 2A disengaged from the distal-most portion of the exemplary retractor;
Fig. 2E illustrates a perspective view of the exemplary removable tip illustrated in Fig. 2A being removed through an incision;
Fig. 3A illustrates a perspective view of an exemplary retractor attached to another exemplary removable tip;
Fig. 3B illustrates a perspective view of the exemplary removable tip illustrated in Fig. 3A in the process of disengagement from the distal-most portion of the exemplary retractor;
Fig. 3C illustrates a perspective view of the exemplary removable tip illustrated in Fig. 3A being disengaged from the distal-most portion of the exemplary retractor;
Fig. 3D illustrates a perspective view of the exemplary removable tip illustrated in Fig. 3A being removed through an incision;
Fig. 4A illustrates a perspective partial cross-sectional view of an exemplary retractor attached to another exemplary removable tip;
Fig. 4B illustrates a perspective view of the exemplary removable tip illustrated in Fig. 4A;
Fig. 4C illustrates a perspective view of the exemplary removable tip illustrated in Fig. 4A just prior to disengagement from the exemplary retractor;
Fig. 4D illustrates a perspective view of the exemplary removable tip illustrated in Fig. 4A disengaged from the exemplary retractor;
Fig. 5A illustrates a perspective view of an exemplary retractor attached to another exemplary removable tip;
Fig. 5B illustrates a perspective view of the exemplary removable tip illustrated in Fig. 5A disengaged from the exemplary retractor;
Fig. 6A illustrates a perspective view of an exemplary retractor attached to another exemplary removable tip;
Fig. 6B is a cross-sectional view of the removable tip taken along line 6A-6A in Fig. 6A;
Fig. 6C illustrates a perspective view of the exemplary removable tip illustrated in Fig. 6A disengaged from the exemplary retractor;
Fig. 7A illustrates a perspective view of an exemplary retractor attached to another exemplary removable tip;
Fig. 7B illustrates a perspective view of the exemplary removable tip illustrated in Fig. 7A disengaged from the exemplary retractor;
Fig. 8A illustrates a perspective view of an exemplary retractor attached to another exemplary removable tip;
Figs. 8B-8E illustrate a perspective view of the exemplary removable tip illustrated in Fig. 8A partially or fully disengaged from the exemplary retractor;
Fig. 9A illustrates a perspective view of an exemplary retractor attached to another exemplary removable tip;
Fig. 9B illustrates a perspective view of the exemplary removable tip illustrated in Fig. 9A in a collapsed configuration;
Figs. 9C-9E illustrate cross-sectional views of the exemplary removable tip illustrated in Fig. 9A showing the collapsing mechanism;
Fig. 10A illustrates a perspective view of an exemplary retractor attached to another exemplary removable tip;
Fig. 10B illustrates a perspective view of the exemplary removable tip illustrated in Fig. 10A in a retracted position;
Figs. 10C-10E illustrate cross-sectional views of the exemplary removable tip illustrated in Fig. 10A comprising various retracting mechanisms;
Fig. 11A illustrates a perspective view of an exemplary retractor attached to another exemplary removable tip;
Fig. 11B illustrates a perspective view of the exemplary removable tip illustrated in Fig. 11A in a deflated configuration;
Figs. 11C-11D illustrate cross-sectional views of the exemplary removable tip illustrated in Fig. 11A further comprising an inflation mechanism;
Fig. 12A illustrates a perspective view of an exemplary retractor attached to another exemplary removable tip;
Fig. 12B illustrates a perspective view of the exemplary removable tip illustrated in Fig. 12A in a partially retracted configuration;
Fig. 13A illustrates a perspective view of an exemplary retractor attached to another exemplary removable tip;
Fig. 13B illustrates a perspective view of the exemplary removable tip illustrated in Fig. 13A disengaged from the exemplary retractor;
Fig. 14A illustrates a perspective view of an exemplary retractor attached to another exemplary removable tip;
Fig. 14B illustrates a perspective view of the exemplary removable tip illustrated in Fig. 14A in a retracted configuration;
Fig. 15A illustrates a perspective view of an exemplary retractor attached to another exemplary removable tip;
Fig. 15B illustrates a perspective view of the exemplary removable tip illustrated in Fig. 15A in a shortened configuration;
Fig. 16A illustrates a perspective partial cross-sectional view of an exemplary retractor attached to another exemplary removable tip;
Fig. 16B illustrates a perspective side view of the distal end of the exemplary removable tip illustrated in Fig. 16A;
Fig. 16C illustrates a perspective view of the exemplary removable tip illustrated in Fig. 16A disengaged from the exemplary retractor; and
Fig. 17 illustrates a method of use of an exemplary retractor-removable tip system, such as those illustrated herein and equivalents thereto.

### DETAILED DESCRIPTION OF PRESENTLY PREFERRED EMBODIMENTS

The exemplary embodiments illustrated provide the discovery of systems, and apparatuses used in conjunction with laparoscopic surgery to improve efficiency and navigation to the target anatomy while maintaining adequate exposure of the target anatomy.

Diseases and conditions contemplated for treatment include, but are not limited to, those involving the pelvic region as well as any other bodily region or field benefiting from improved navigation to a target site.

The present invention is not limited to those embodiments illustrated herein, but rather, the disclosure includes all equivalents including those of different shapes, sizes, and configurations. The systems, devices, and methods may be used in any field benefiting retractors or devices to aid in the navigation to a target site. Additionally, the devices and methods are not limited to being used with human beings; others are contemplated, including but not limited to, animals.

Unless otherwise defined, all technical and scientific terms used herein have the same meaning as commonly understood by one of ordinary skill in the art. In case of conflict, the present document, including definitions, will control. Preferred methods and materials are illustrated below, although apparatuses, methods, and materials similar or equivalent to those illustrated herein may be used in practice or testing. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The present disclosure also contemplates other embodiments "comprising," "consisting of" and "consisting essentially of," the embodiments or elements presented herein, whether explicitly set forth or not.

The term "proximal," as used herein, refers to a direction that is generally towards a physician during a medical procedure.

The term "distal," as used herein, refers to a direction that is generally towards a target site within a patient's anatomy during a medical procedure.

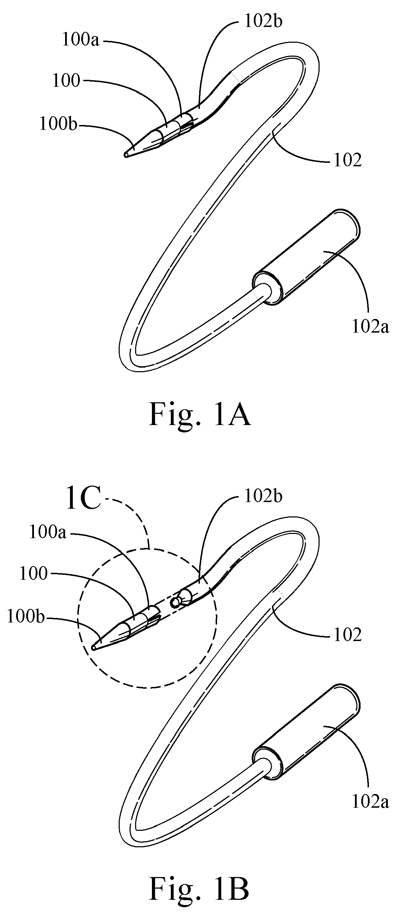

A more detailed description of the embodiments will now be given with reference to Figs. 1A-17. Throughout the disclosure, like reference numerals and letters refer to like elements. The present disclosure is not limited to the embodiments illustrated; to the contrary, the present disclosure specifically contemplates other embodiments not illustrated but intended to be included in the claims.

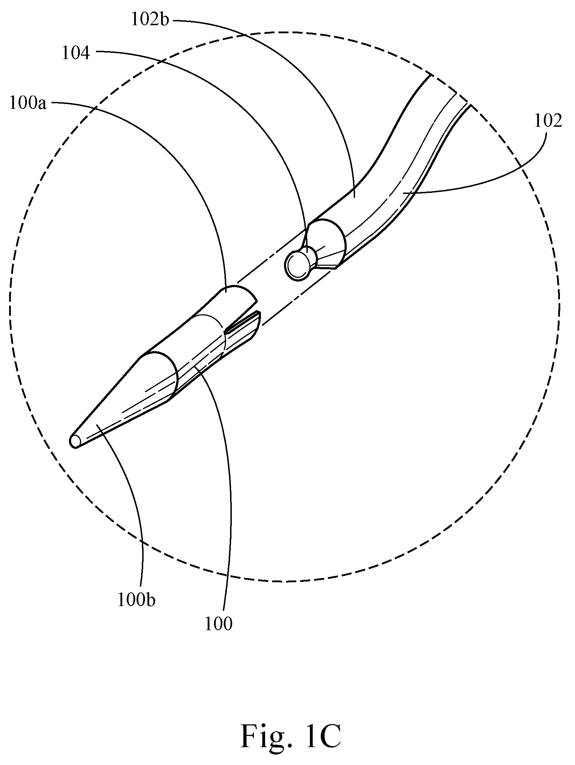

Fig. 1A illustrates a perspective view of exemplary retractor 102 attached to exemplary removable tip 100, Fig. 1B illustrates a perspective view of exemplary retractor 102 detached from exemplary removable tip 100, and Fig. 1C illustrates a perspective view of a portion of exemplary retractor 102 detached from exemplary removable tip 100 illustrated in Fig. 1B at the circle 1C. Referring to Fig. 1A, exemplary retractor 102 includes proximal portion 102a and distal portion 102b. The distal-most portion of retractor 102 has been modified to receive a female proximal portion 100a of removable tip 100. Retractor 102 is designed for insertion through a laparoscopic incision or port and is preferably used to aid in the positioning and or holding of organs or tissues during a diagnostic, exploratory, or therapy procedure. For example, retractor 102 may be used to help position organs and tissues during, for example, rectal dissections, pelvic floor grafting, uterine procedures, and bowel procedures. Moreover the use of two or more retractors 102 having removable tips 100 are contemplated.

The distal-most end of retractor 102 is atraumatic, pinched, and tapered. Proximal portion 100a of removable tip 100 is substantially hollow to receive retractor 102 via a snap fit or other attachment means, including but not limited to, a friction fit. A screw/threaded attachment means may also be used, recognizing that such an attachment means may be more difficult to disengage when in use due to the space constraints of the working environment.

Removable tip 100 is a substantially conical-shaped removable piece configured for attachment to distal portion 102b of retractor 102 to improve retractor 102 insertion, especially in the case of patients having large amounts of adipose tissue. Distal portion 100b of removable tip 100 is tapered and atraumatic for ease of insertion and navigation through an incision and to prevent damaging organs and tissue. Although removable tip 100 is illustrated as being configured to receive retractor 102 therein, retractor 102 may be configured to receive removable tip 100 therein. In other words, retractor 102 may be configured with a female distal end 102b into which a male proximal end 100a of removable tip 100 may be inserted and affixed thereto. In any event, in either configuration, it is generally preferred, although not required, that a medical retractor be free from numerous crevasses or other surface features that may make sterilization for reuse difficult.

The additional overall length provided by removable tip 100 makes it easier for retractor 102 to be inserted through the incision during a laparoscopic procedure. The longer the removable tip 100, the more adipose tissue can be accommodated. In other words, fashioning removable tip 100 to have a longer dimension will allow it to accommodate thicker skin, fat, and muscle layers. However, because removable tip 100 is removable from retractor 102 after retractor 102 is inserted, the space constraints addressed by a retractor 102 having a minimized overall size may be achieved.

Referring to Figs. 1B and 1C, removable tip 100 is illustrated as disengaged from retractor 102 by being pulled distally from retractor 102, using, for example, an instrument such as endo grippers. Once removable tip 100 is disengaged from retractor 102, retractor 102 alone is of a sufficient length to perform its retracting functions. The distal-most portion of retractor 102 includes a tapered atraumatic portion 104 for ease of insertion through an incision or port that will not damage tissue or organs once disengaged from removable tip 100.

Removable tip 100 is approximately 5 cm long but other lengths are contemplated depending upon the amount of tissue needed through which to introduce retractor 102. For example, removable tips 100 having a variety of lengths may be manufactured to accommodate various amounts of adipose or other tissues and could be provided as part of a kit. For example, a first removable tip 100 may have a length of 5 cm, a second may have a length of 8 cm, and a third may have a length of 10 cm. Accordingly, the user may choose the most appropriately-sized removable tip 100 for attachment to retractor 102 based on the clinical facts and circumstances. In some cases, if the material through which retractor 102 needs to be inserted is sufficiently thin, removable tip 100 may not be needed.

Removable tip 100 is made from plastic, such as polypropylene, although other materials are contemplated, including but not limited to TPE (thermoplastic elastomers), polycarbonate, polystyrene, an elastomeric material capable of stretching over the distal-most portion of retractor 102, molded silicone, and metal. Removable tip 100 may be configured from two or more materials, including those having different properties, characteristics or features, including but not limited to, one material having a different hardness from the other. For example, distal portion 100b of removable tip 100 may be configured from a harder material than, for example, proximal portion 100a of removable tip 100. The harder material may provide the distal portion 100b with an improved ability to pass through the tissue during placement, whereas the softer material may provide the proximal portion 100a with an improved ability to engage with the distal end 102b of the retractor 102.

Removable tip 100 preferably is disposable, low-cost, and is intended for single use, although it may be configured to be reusable. Retractor 102 is preferably also low-cost and may be manufactured for either single use or for multiple uses.

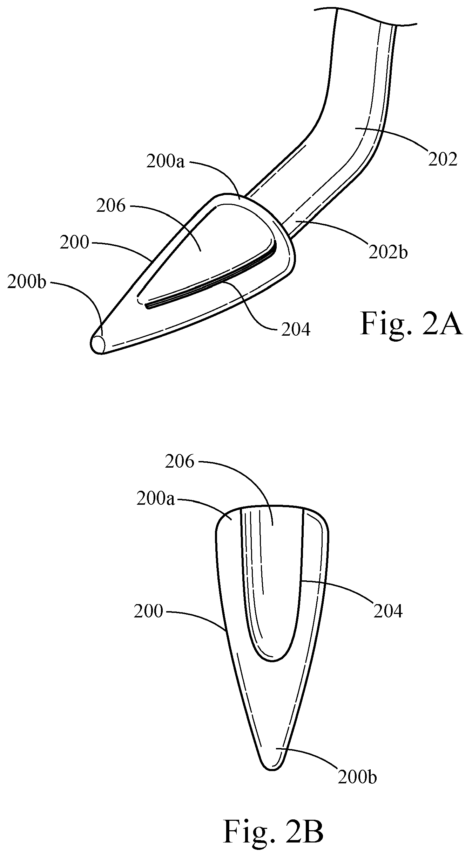

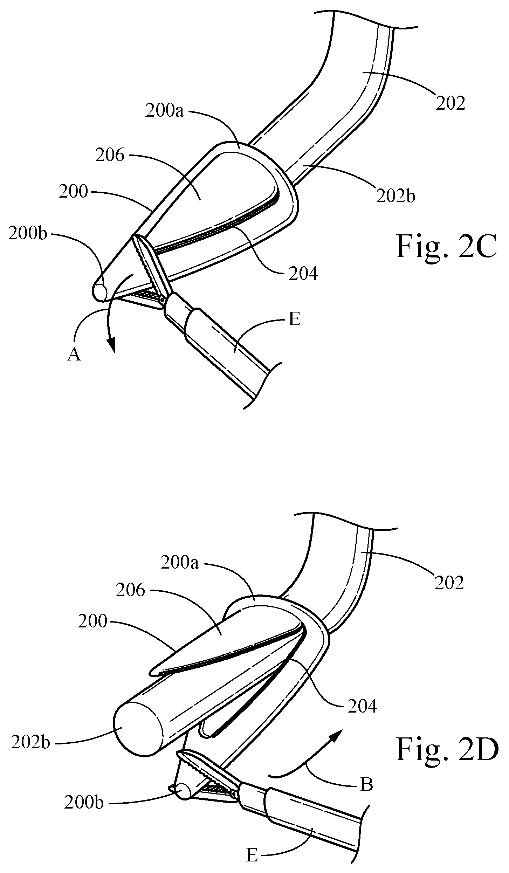

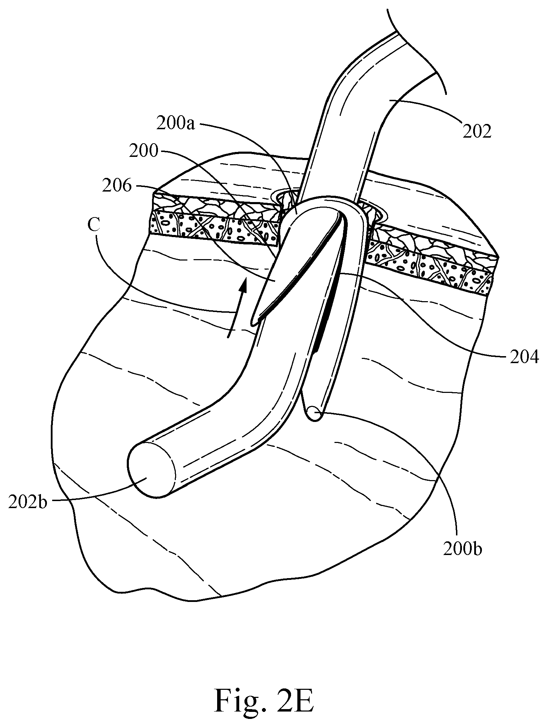

Fig. 2A illustrates a perspective view of exemplary retractor 202 attached to exemplary removable tip 200, Fig. 2B illustrates a bottom perspective view of removable tip 200, Fig. 2C illustrates a perspective view of removable tip 200 just prior to disengagement from retractor 202, Fig. 2D illustrates a perspective view of removable tip 200 disengaged from the distal-most portion of retractor 202, and Fig. 2E illustrates a perspective view of removable tip 200 being removed through an incision. Referring to Figs. 2A-2B, retractor 202 is similar to the retractor embodiment discussed above in connection with Figs. 1A-1C. The distal-most end of retractor 202 is atraumatic and rounded. For example, retractor 202 is designed for insertion through a laparoscopic incision or port and is preferably used to aid in the positioning and or holding of organs or tissues during a diagnostic, exploratory, or therapy procedure. For example, retractor 202 may be used to help position organs and tissues during, for example, rectal dissections, pelvic floor grafting, uterine procedures, and bowel procedures. Although only a single retractor 202 is illustrated, the use of two or more retractors 202 having removable tips 200 is also contemplated.

Removable tip 200 is a substantially conical-shaped removable piece configured for attachment to distal portion 202b of retractor 202 to improve the insertion capability of retractor 202, especially in the case of patients having large amounts of adipose tissue. Distal portion 200b of removable tip 200 is tapered and atraumatic for ease of insertion and navigation through an incision, as well as to prevent causing damage to organs and tissue. Proximal portion 200a of removable tip 200 is substantially hollow so as to receive retractor 202 therein. As will be explained below, removable tip 200 is configured to be disengaged from retractor 202 by breaking the removable tip 200 at snap point 204. For example, referring to Fig. 2C, an instrument, such as a forceps or endo gripper E is positioned at and grabs distal portion 200b of removable tip 200. Endo gripper E is then moved in the direction of arrow A to cause distal portion 200b of removable tip 200 to break from supporting neck 206 at snap point 204, thereby releasing the engagement of removable tip 200 with retractor 202. Removable tip 200 includes supporting neck 206 that maintains, at least temporarily, communication with retractor 202 even after distal portion 200b of removable tip 200 is broken at snap point 204.

Referring to Fig. 2D, an instrument, such as endo grippers E is used to back feed removable tip 200 proximally along retractor 202 in the direction of arrow B such that removable tip 200 is able to be removed from the area of the laparoscopic procedure. Referring to Fig. 2E, removable tip 200 continues to be back-thread proximally along retractor 202 in the direction of arrow C such that it is pushed through the incision point at the skin and the abdominal cavity, and is subsequently removed from the procedure. Once removable tip 200 is disengaged from retractor 202, retractor 202 alone is of a sufficient length to perform its retracting functions.

Removable tip 200 is approximately 5 cm long but other lengths are contemplated depending upon the amount of tissue through which retractor 202 is to be introduced. For example, multiple removable tips 200 each having a variety of lengths may be manufactured to accommodate various amounts of adipose or other tissues. For example, a first removable tip 200 may have a length of 5 cm, a second may have a length of 8 cm, and a third may have a length of 10 cm. The removable tips 200 having various lengths may be included in a kit supplied with retractor 202. Accordingly, the user may choose the most appropriately-sized removable tip 200 for attachment to retractor 202 based on the clinical facts and circumstances. In some cases, the material through which retractor 202 needs to be inserted may be sufficiently thin such that removable tip 200 may not be needed.

Removable tip 200 is made from plastic, such as polypropylene, although other materials are contemplated, including but not limited to TPE (thermoplastic elastomers), polycarbonate, polystyrene, nylon, and molded silicone. Removable tip 200 may be configured from two or more materials, including those having different properties, characteristics or features, including but not limited to, one having a different hardness from the other. For example, distal portion 200b of removable tip 200 may be configured from a harder material than, for example, proximal portion 200a of removable tip 200.

Removable tip 200 preferably is disposable, low-cost, and is intended for single use, although it may be configured to be reusable. Retractor 202 is preferably also low-cost and may be manufactured for single use or for multiple uses.

One advantage of the configuration of removable tip 200, among many, is that removable tip 200 is never completely disengaged from the retractor 202 while within the patient. Accordingly, there is very little risk of removable tip 200 becoming lost or forgotten within the patient.

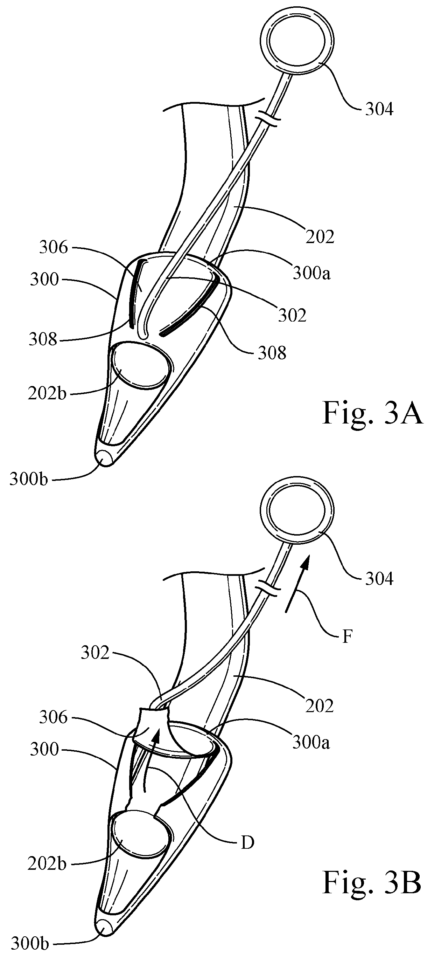

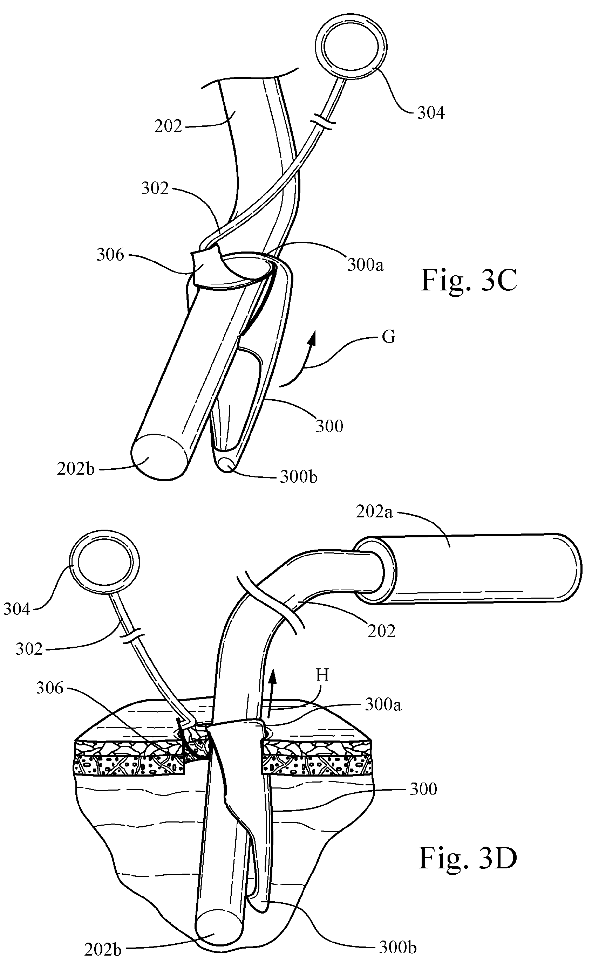

Fig. 3A illustrates a perspective view of exemplary retractor 202 attached to exemplary removable tip 300, Fig. 3B illustrates a perspective view of removable tip 300 in the process of disengagement from the distal-most portion of retractor 202, Fig. 3C illustrates a perspective view of removable tip 300 being disengaged from the distal-most portion of retractor 202, and Fig. 3D illustrates a perspective view of removable tip 300 being removed through an incision. Although a single retractor 202 is illustrated, the use of two or more retractors 202 each having removable tips 300 is contemplated.

Removable tip 300 includes proximal portion 300a and distal portion 300b. Removable tip 300 is a substantially conical-shaped removable piece configured for attachment to distal portion 202b of retractor 202 to improve retractor insertion, especially in the case of patients having large amounts of adipose tissue. Referring to Fig. 3A, proximal portion 300a of removable tip 300 is substantially hollow and configured for receiving distal portion 202b of retractor 202, via, for example, a snap fit or other attachment means, including but not limited to a friction fit. Distal portion 300b of removable tip 300 is tapered and atraumatic for ease of insertion and navigation through an incision and to prevent damaging organs and tissue. In this particular embodiment, removable tip 300 includes pull cord 302 connected to ring 304 that when pulled, breaks removable tip 300 at perforations 308, thereby causing removable tip 300 to release its grip on retractor 202 such that removable tip 300 may be removed. Ring 304 or other retaining means is intended to remain outside patient while retractor 202 equipped with removable tip 300 is being inserted into a patient.

Referring to Figs. 3B and 3C, ring 304 is pulled proximally in the direction of arrow F thereby causing perforations 308 (illustrated in Fig. 3A) to break and create break flap 306 in the direction of arrow D. The entirety of tip 300 is then pulled proximally along retractor 202 in the direction of arrow G. Referring to Fig. 3D, removable tip 300 continues to be back-thread proximally along retractor 202 in the direction of arrow H such that it is pushed through the incision point at skin and the abdominal cavity and is able to be removed from the procedure. Once removable tip 300 is disengaged from retractor 202, retractor 202 alone is of a sufficient length to perform its retracting functions.

Removable tip 300 is approximately 15 cm long but other lengths are contemplated depending upon the amount of tissue needed through which to introduce retractor 202. For example, multiple removable tips 300 having a variety of lengths may be manufactured to accommodate various amounts of adipose or other tissues. For example, a first removable tip 300 may have a length of 15 cm, another may have a length of 18 cm, and another may have a length of 20 cm. Accordingly, the user may choose the most appropriately-sized removable tip 300 for attachment to retractor 202 based on the clinical facts and circumstances. In some cases, if the material through which retractor 202 needs to be inserted is sufficiently thin, removable tip 300 may not be needed.

Removable tip 300 is made from plastic, such as polypropylene, although other materials are contemplated, including but not limited to TPE (thermoplastic elastomers), polycarbonate, polystyrene, nylon, and molded silicone. Removable tip 300 may be configured from two or more materials, including those having different characteristics or features, including but not limited to, one having a different hardness from the other. For example, distal portion 300b of removable tip 300 may be configured from a harder material than, for example, proximal portion 300a of removable tip 300.

Removable tip 300 preferably is disposable, low-cost, and is intended for single use, although it may be configured to be reusable. One advantage of the configuration of removable tip 300, among many, is that removable tip 300 is never completely disengaged from retractor 202 while within the patient. Accordingly, there is very little risk of removable tip 300 becoming lost or forgotten within the patient.

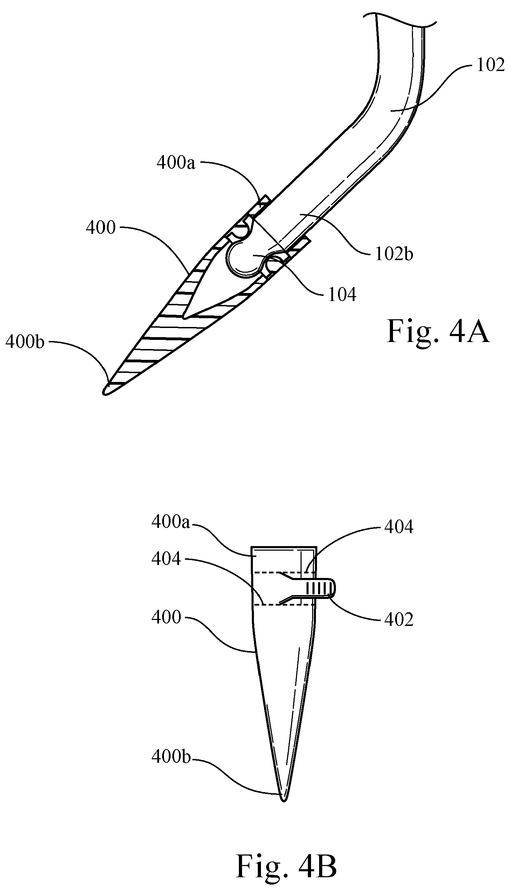

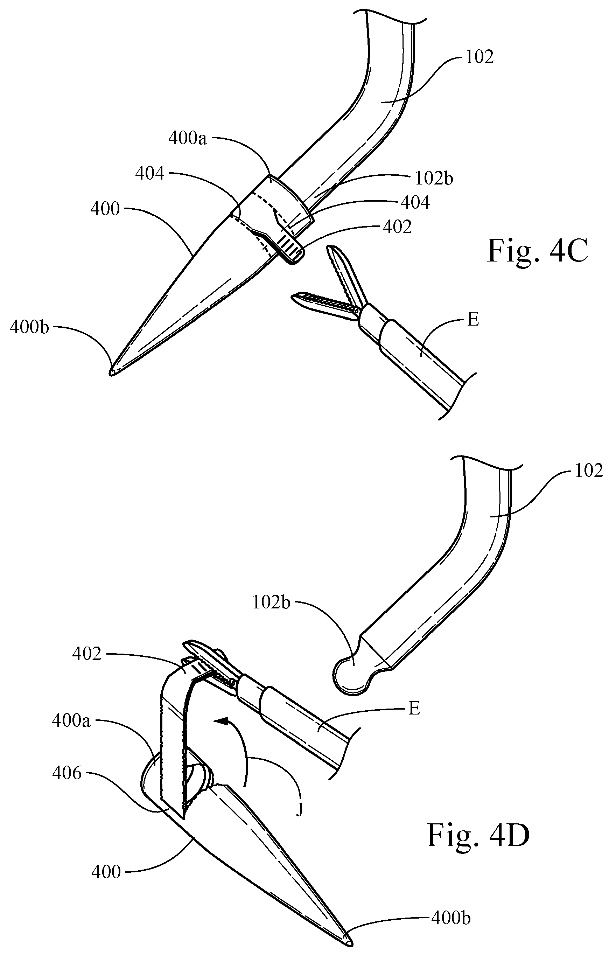

Fig. 4A illustrates a perspective partial cross-sectional view of exemplary retractor 102 attached to exemplary removable tip 400, Fig. 4B illustrates a perspective view of removable tip 400, Fig. 4C illustrates a perspective view of removable tip 400 just prior to disengagement from the exemplary retractor, and Fig. 4D illustrates a perspective view of removable tip 400 disengaged from the exemplary retractor. The use of two or more retractors 102 having removable tips 400 are contemplated.

Referring to Figs. 4A and 4B, removable tip 400 includes proximal portion 400a and distal portion 400b. Removable tip 400 is a substantially conical-shaped removable piece configured for attachment to distal portion 102b of retractor 102 to improve retractor insertion, especially in the case of patients having large amounts of adipose tissue. Distal portion 400b of removable tip 400 is tapered and atraumatic for ease of insertion and navigation through an incision and to prevent damaging organs and tissue. Proximal portion 400a of removable tip 400 is substantially hollow and configured for receiving distal portion 102b of retractor 102, via, for example, a snap fit or other attachment means, including but not limited to a friction fit. In the particular embodiment illustrated, the distal portion 102b of the retractor 102 comprises one or more grooves or indentations that are configured to receive one or more ridges or protrusion on the inside of removable tip 400 to facilitate engagement between therebetween. Removable tip 400 includes pull tab 402 that when pulled, breaks removable tip 400 at perforations 404, thereby causing removable tip 400 to release its grip on retractor 102 such that removable tip 400 may be removed.

Referring to Figs. 4C and 4D, an instrument, such as a forceps or an endo gripper E is positioned at and grasps pull tab 402. The endo gripper E pulls pull tab 402 in the direction of arrow J away from removable tip 400, thereby causing perforations 404 to break until perforation stop 406 such that removable tip 400 remains one piece. Removable tip 400 is disengaged from retractor 102 and can be removed through any incision or port, or after the procedure is complete. Once removable tip 400 is disengaged from retractor 102, retractor 102 alone is of a sufficient length to perform its retracting functions.

Removable tip 400 is approximately 5 cm long but other lengths are contemplated depending upon the amount of tissue needed through which to introduce retractor 102. For example, multiple removable tips 400 having a variety of lengths may be manufactured to accommodate various amounts of adipose or other tissues. For example, a first removable tip 400 may have a length of 5 cm, another may have a length of 8 cm, and another may have a length of 10 cm. Accordingly, the user may choose the most appropriately-sized removable tip 400 for attachment to retractor 102 based on the clinical facts and circumstances. In some cases, if the material through which retractor 102 needs to be inserted is sufficiently thin, removable tip 400 may not be needed.

Removable tip 400 is made from plastic, such as polypropylene, although other materials are contemplated, including but not limited to TPE (thermoplastic elastomers), polycarbonate, polystyrene, nylon, and molded silicone. Removable tip 400 may be configured from two or more materials, including those having different characteristics or features, including but not limited to, one having a different hardness from the other. For example, distal portion 400b of removable tip 400 may be configured from a harder material than, for example, proximal portion 400a of removable tip 400. Removable tip 400 preferably is disposable, low-cost, and is intended for single use, although it may be configured to be reusable.

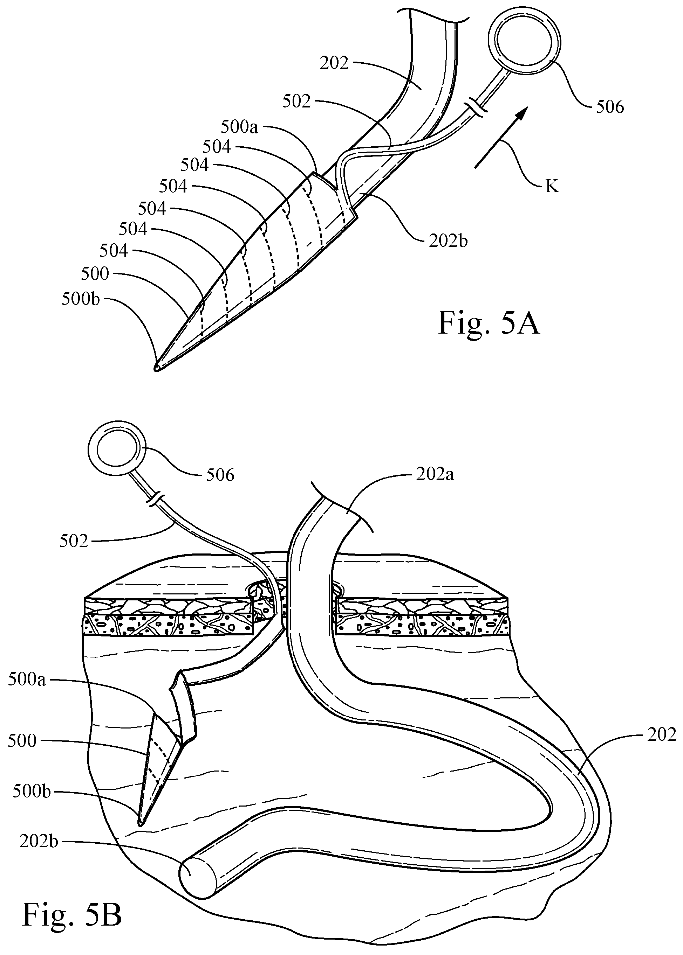

Fig. 5A illustrates a perspective view of exemplary retractor 202 attached to exemplary removable tip 500, and Fig. 5B illustrates a perspective view of removable tip 500 disengaged from exemplary retractor 202. The use of two or more retractors 202 having removable tips 500 are contemplated.

Removable tip 500 includes proximal portion 500a and distal portion 500b. Removable tip 500 is a substantially conical-shaped removable piece configured for attachment to distal portion 202b of retractor 202 to improve retractor insertion, especially in the case of patients having large amounts of adipose tissue. Distal portion 500b of removable tip 500 is tapered and atraumatic for ease of insertion and navigation through an incision and to prevent damaging organs and tissue. Proximal portion 500a of removable tip 500 is substantially hollow and configured for receiving distal portion 202b of retractor 202.

Removable tip 500 includes pull cord 502 connected to ring 506 that when pulled, breaks removable tip 500 at spiral perforations 504 disposed about removable tip 500, thereby causing removable tip 500 to unwind and release its grip on retractor 202 such that removable tip 500 may be removed. Ring 506 or other retaining means is intended to remain outside patient while retractor 202 equipped with removable tip 500 is being inserted into a patient.

Ring 506 is pulled proximally in the direction of arrow K, thereby causing perforations 504 to break and unravel tip 500. The entirety of tip 500 is then pulled out of the way from the area of retractor 202. For example, ring 506 or any portion of pull cord 502 may be taped, clipped, or adhered, to an article such as the skin's surface, to maintain the placement of removable tip 500. Once removable tip 500 is disengaged from retractor 202, retractor 202 alone is of a sufficient length to perform its retracting functions. After retractor 202 is removed from the incision site, removable tip 500 is likewise able to be removed from the incision site.

One advantage of the configuration of removable tip 500, among many, is that removable tip 500 is always connected to retractor 202 or the outside surface of the patient during the procedure. Accordingly, there is very little risk of removable tip 500 becoming lost or forgotten within the patient.

Removable tip 500 is approximately 15 cm long but other lengths are contemplated depending upon the amount of tissue needed through which to introduce retractor 202. For example, multiple removable tips 500 having a variety of lengths may be manufactured to accommodate various amounts of adipose or other tissues and provided with retractor 202 as a kit. For example, a first removable tip 500 may have a length of 15 cm, another may have a length of 18 cm, and another may have a length of 20 cm. Accordingly, the user may choose from the kit the most appropriately-sized removable tip 500 for attachment to retractor 202 based on the clinical facts and circumstances. In some cases, if the material through which retractor 202 needs to be inserted is sufficiently thin, removable tip 500 may not be needed.

Removable tip 500 is made from plastic, such as polypropylene, although other materials are contemplated, including but not limited to TPE (thermoplastic elastomers), polycarbonate, polystyrene, nylon, and molded silicone. Removable tip 500 may be configured from two or more materials, including those having different properties, characteristics or features, including but not limited to, one having a different hardness from the other. For example, distal portion 500b of removable tip 500 may be configured from a harder material than, for example, proximal portion 500a of removable tip 500. Removable tip 500 preferably is disposable, low-cost, and is intended for single use, although it may be reusable.

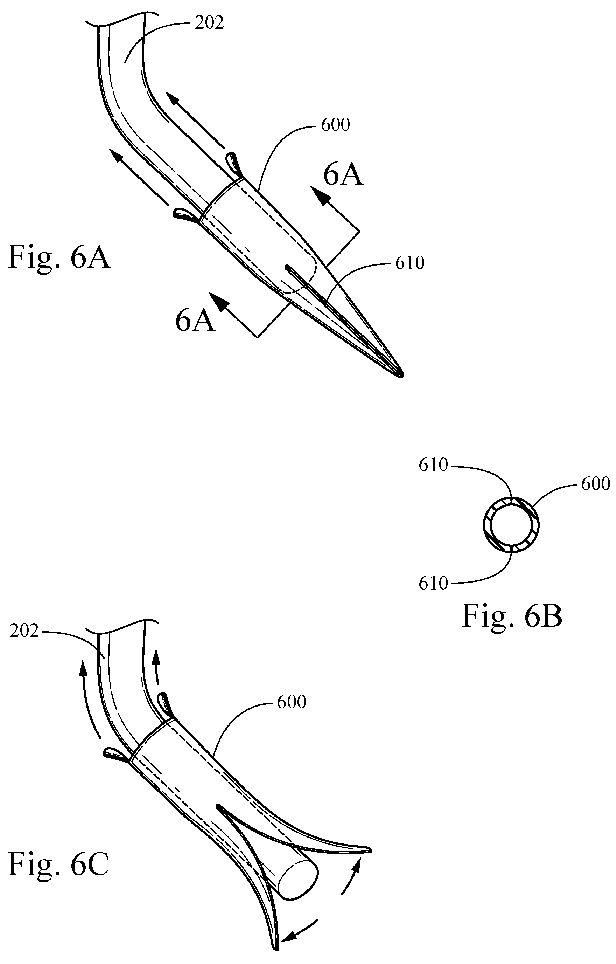

Figs. 6A-6C illustrate another embodiment of a removable tip 600 for use with retractor 202. In this particular embodiment, the removable tip 600 comprises a pair of break lines 610 disposed longitudinally along the distal portion thereof. The break lines 610 are configured to break apart as the removable tip 600 is pulled in a proximal direction to thereby allow the removable tip 600 to be retracted in a proximal direction along the retractor 202. Pull tabs may be provided on the proximal end of the removable tip 600 to facilitate the application of the breaking force and removal of the removable tip 600.

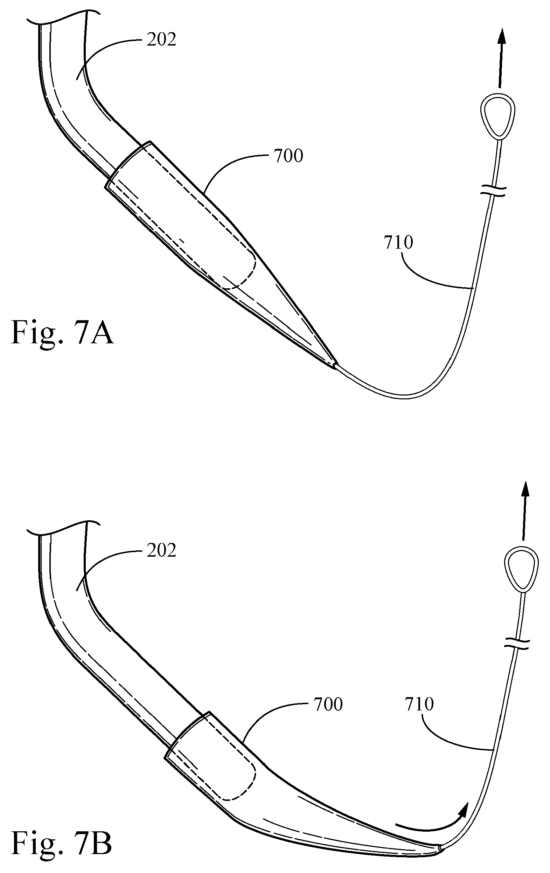

Figs. 7A-7B illustrate another embodiment of a removable tip 700 for use with retractor 202. In this particular embodiment, the removable tip 700 includes a pull cable 710 attached to the distal end the removable tip 700. Once the retractor 202 is position, the user pulls on the pull cable 710 with sufficient force to separate and remove the removable tip 700 from the retractor 202. The distal end of the pull cable 710 may include a loop to facilitate grasping thereof.

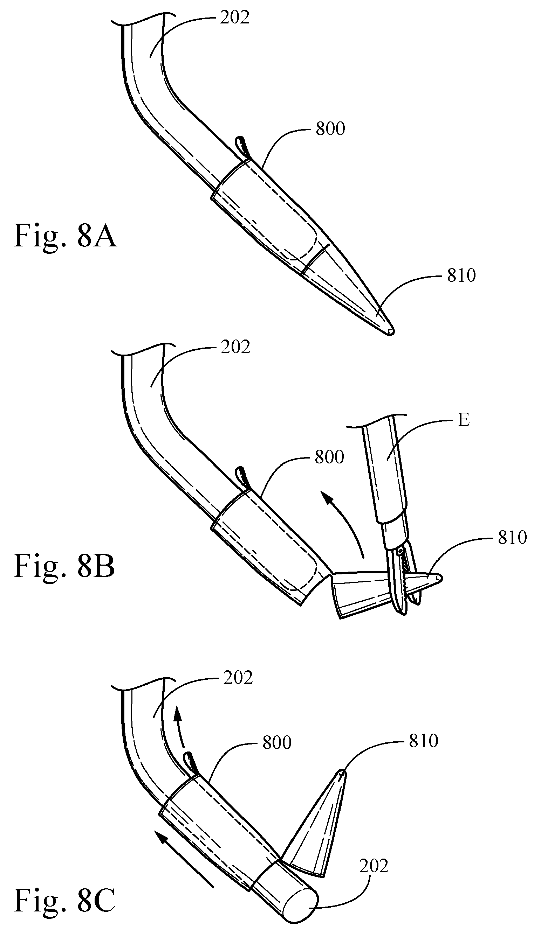

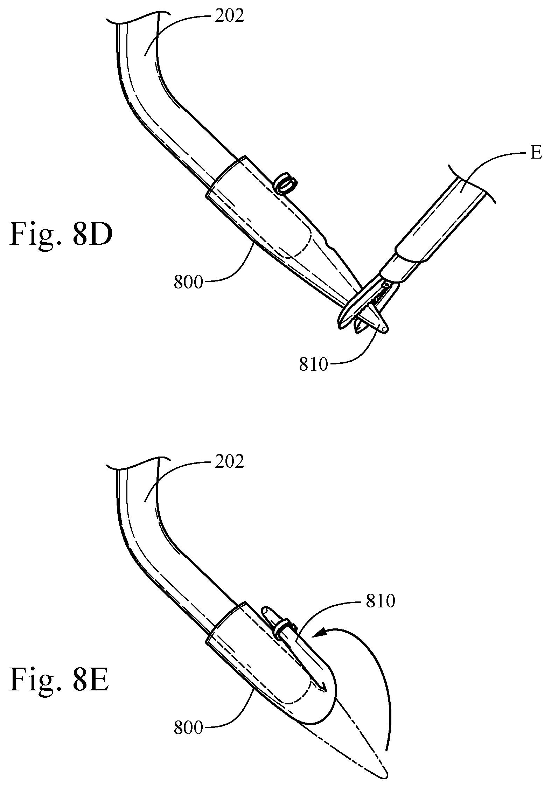

Figs. 8A-8E illustrate another embodiment of a removable tip 800 for use with a retractor 202. In this particular embodiment, the removable tip 800 comprises a foldable distal portion 810 that may be folded to either permit removal or reduce the overall length thereof. Referring to Fig. 8B, a forceps or endo grasper E is used to fold the distal portion 810. As show in Fig. 8C, the folding of the distal portion 810 exposes an opening in the removable tip 800, thereby permitting the removable tip 800 to be retracted proximally along the retractor 202. In the variation illustrated in Figs. 8D-8E, the foldable distal portion 810 is folded and secured alongside the remaining portion of the removable tip 800, thereby reducing the overall length thereof.

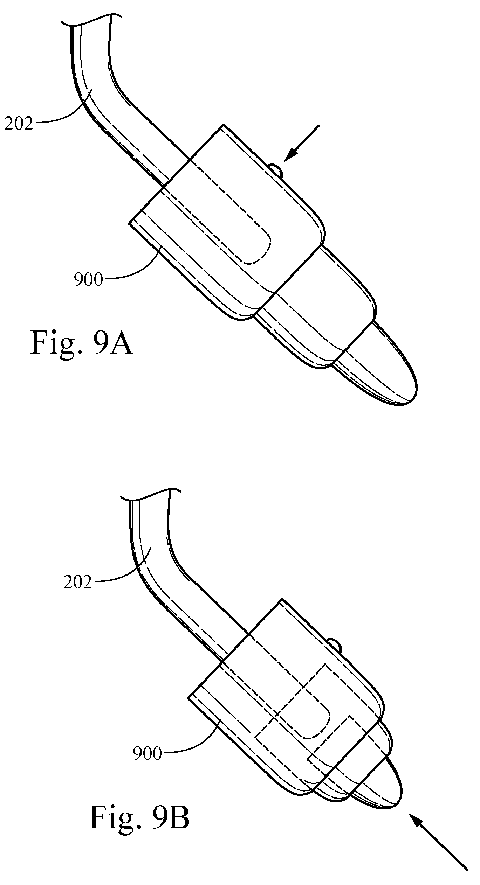

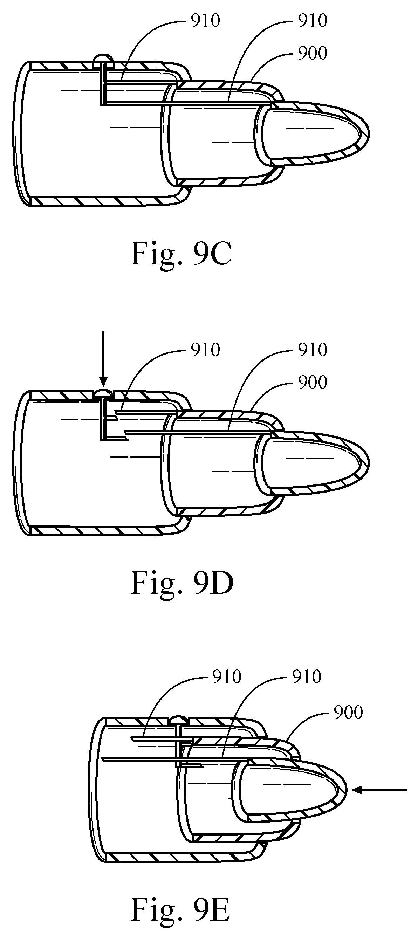

Figs. 9A-9E illustrate another embodiment of a removable tip 900 for use with a retractor 202. In this particular embodiment, the removable tip 900 comprises a collapsing mechanism 910 that permits the overall length of the removable tip 900 to be reduced. In particular, and as best seen in Figs. 9C-9E, the collapsing mechanism 910 comprises a plurality of interior supports that are secured to telescoping portions of the removable tip 900. A button or other activation device on the removable tip 900 is depressed to break the interior supports, thereby allowing the telescoping sections to be collapsed, as shown in Fig. 9B.

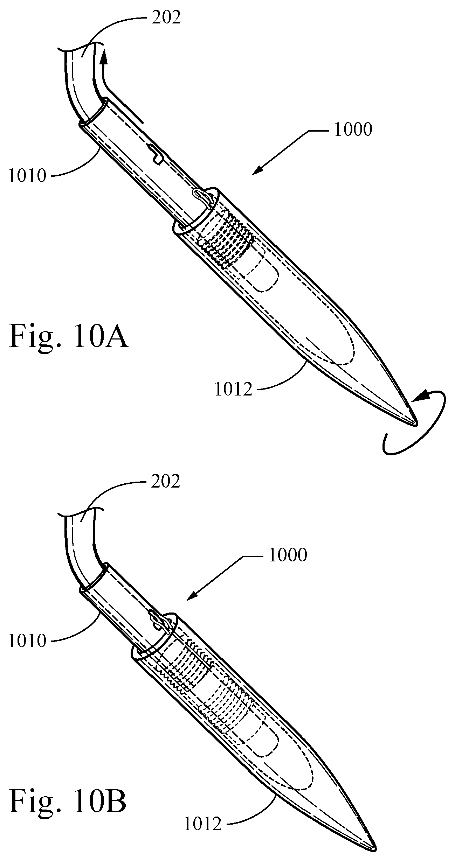

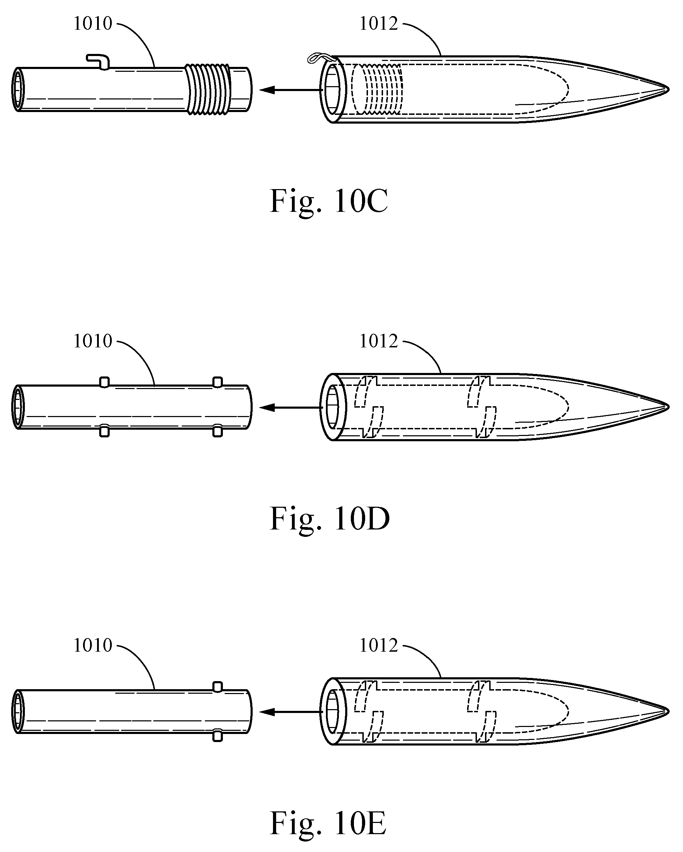

Figs. 10A-10E illustrate another embodiment of a removable tip 1000 for use with a retractor 202. In this particular embodiment, the removable tip 1000 comprises a retracting mechanism that permits the overall length of the removable tip 1000 to be reduced. With reference to Figs. 10A-10C, the retracting mechanism includes a screw thread disposed on the outer surface of proximal portion 1010 which is configured to mate with a screw thread on the internal surface of distal portion 1012. Rotation of the distal portion 1012 relative to the proximal portion 1010 causes the distal portion to move in a proximal direction relative to the retractor 202, thereby allowing the overall length of the removable tip 1000 to be reduced. Figs. 10D-10E illustrate an alternative arrangement wherein a bayonet connection is used in lieu of the screw connection. In the particular embodiments illustrated, the proximal portion 1010 is a separate component that is affixed to the distal end of the retractor 202. However, it should be understood that the proximal portion 1010 could be integrally formed with or a formed into the distal end of the retractor 202.

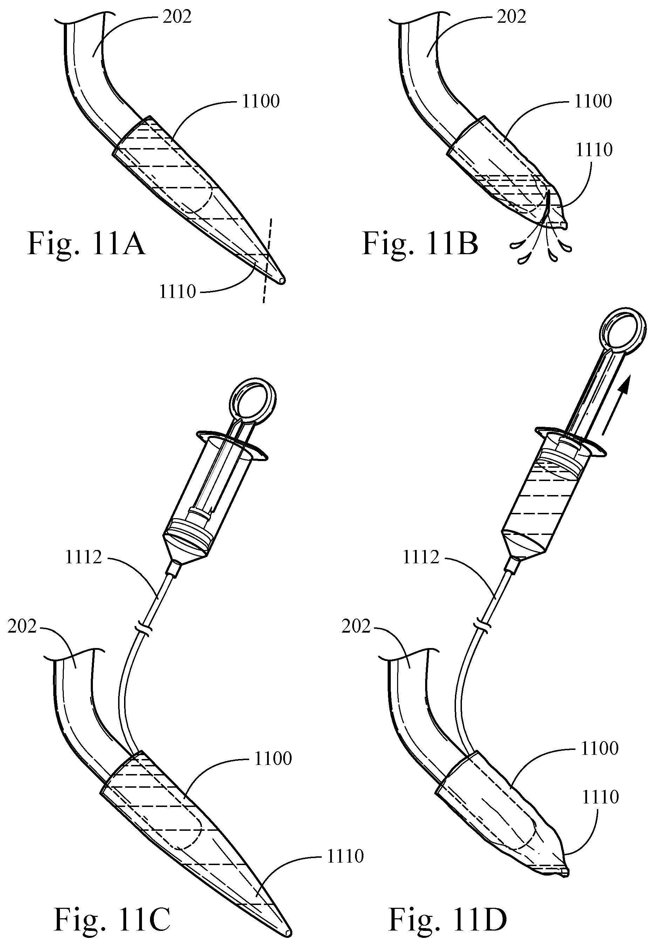

Figs. 11A-11D illustrate another embodiment of a removable tip 1100 for use with a retractor 202. In this particular embodiment, the removable tip 1100 comprises an inflatable balloon tip 1110 that is filled with saline. The saline is removed from the balloon tip 1110 to deflate and collapse the balloon tip 1110, thereby reducing the overall length of the removable tip 1100. With reference to Fig. 11B, the balloon tip 1110 is cut or punctured to allow the saline to escape. With reference to Figs. 11C-11D, the removable tip 1100 further includes an inflation mechanism 1112 for delivering saline to or removing saline from the balloon tip 1110. The balloon tip 1110 could comprise multiple chambers to allow removable tip 1100 to be inflated to various lengths or configuration.

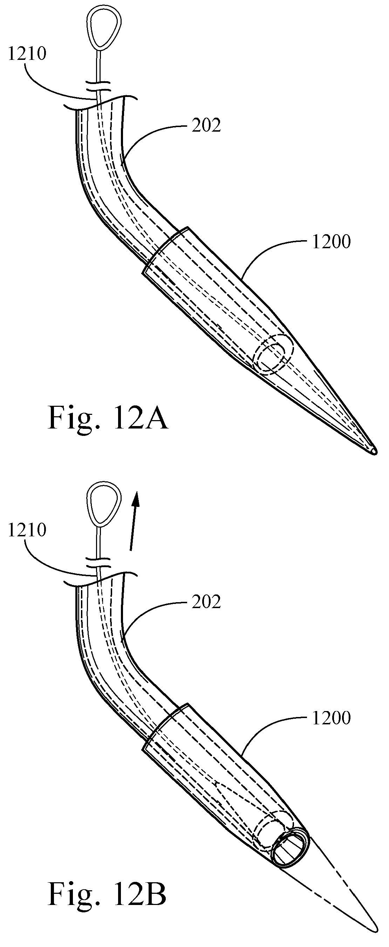

Figs. 12A-12B illustrate another embodiment of a removable tip 1200 for use with a retractor 202. In this particular embodiment, the removable tip 1200 comprises a deformable and/or compressible material. As shown in Fig. 12A, a pull cable 1210 is attached the distal end of the removable tip 1200. The pull cable 1210 passes proximally through a lumen or hollowed out portion of the removable tip 1200, and then proximally through a lumen of the retractor 202. Once the retractor 202 is in position, the used pulls the pull cable 1210 to invert the distal end of the removable tip 1200. The distal end of the removable tip 1200 is then pulled back into the lumen or hollowed out portion of the removable tip 1200, thereby reducing the overall length of the removable tip 1200. In the particular embodiment illustrated, the distal portion of the removable tip 1200 is also pulled into the lumen of the retractor 202. If the removable tip 1200 comprises a sufficiently compressible material, the removable tip may be pulled completely into the lumen of the retractor 202.

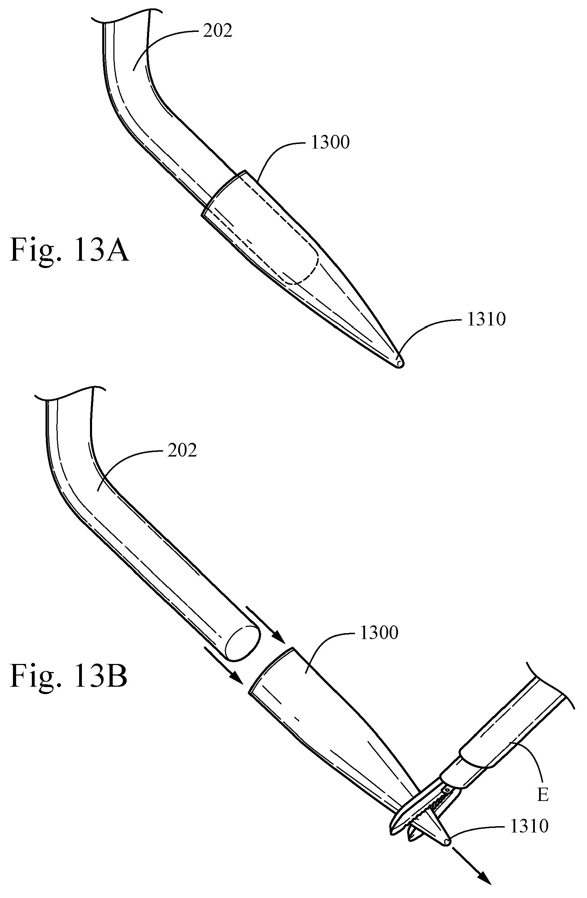

Figs. 13A-13B illustrate another embodiment of a removable tip 1300 for use with retractor 202. In this particular embodiment, the removable tip 1300 includes a grasping mechanism 1310 attached to the distal end the removable tip 1300. Once the retractor 202 is position, the user grasps the grasping mechanism 1310 with a forceps or endo grasper E with sufficient force to separate and remove the removable tip 1300 from the retractor 202. In the particular embodiment illustrated, the grasping mechanism 1310 comprises a port in the distal end of the removable tip 1300. However, other grasping mechanisms are contemplated, such a wire ring or suture loop.

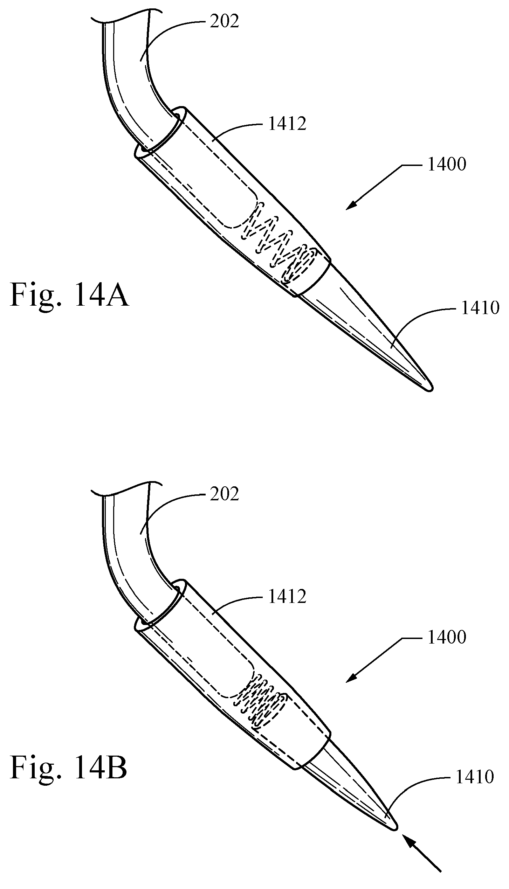

Figs. 14A-14B illustrate another embodiment of a removable tip 1400 for use with a retractor 202. In this particular embodiment, the removable tip 1400 comprises a spring loaded retracting mechanism that permits the overall length of the removable tip 1400 to be reduced. In particular, the removable tip 1400 comprises a distal portion 1410 that is slidably coupled to a proximal portion 1412, wherein a coil spring is disposed therebetween. Once the retractor 202 is in position, pressure is applied to the distal portion 1410 to cause the distal portion 1410 to move in a proximal direction relative to the proximal portion 1412 and the retractor 202, thereby allowing the overall length of the removable tip 1400 to be reduced, as illustrated in Fig. 14B. A detent or similar mechanism may be provided between the distal portion 1410 and the proximal portion 1412 to secure the removable tip 1400 in a collapsed configuration. In the particular embodiments illustrated, the proximal portion 1412 is a separate component that is affixed to the distal end of the retractor 202. However, it should be understood that the proximal portion 1412 could be integrally formed with or a formed into the distal end of the retractor 202.

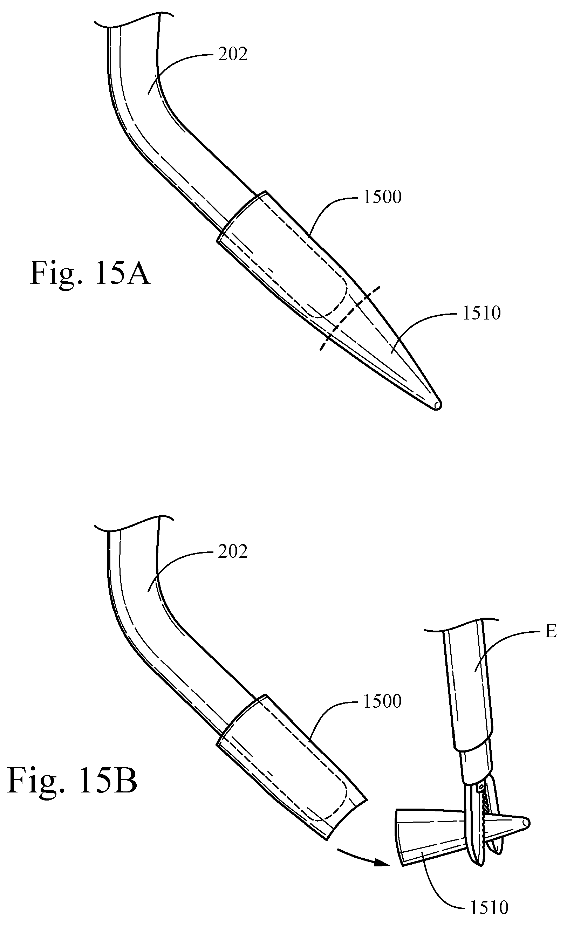

Figs. 15A-15B illustrate another embodiment of a removable tip 1500 for use with a retractor 202. In this particular embodiment, the removable tip 1500 comprises a distal end portion 1510 that is removable. In particular, the distal end portion 1510 is cut and removed from the removable tip 1500 once the retractor 202 is in position. The distal end portion 1510 may also be snapped off and removed. To aid in the removal of the distal end portion 1510, the removable tip 1500 may comprise a weakened area configured to facilitate removal of the distal end portion 1510. Once removed, a forceps or endo grasper E may be used to remove the distal end portion 1510 from the surgical site.

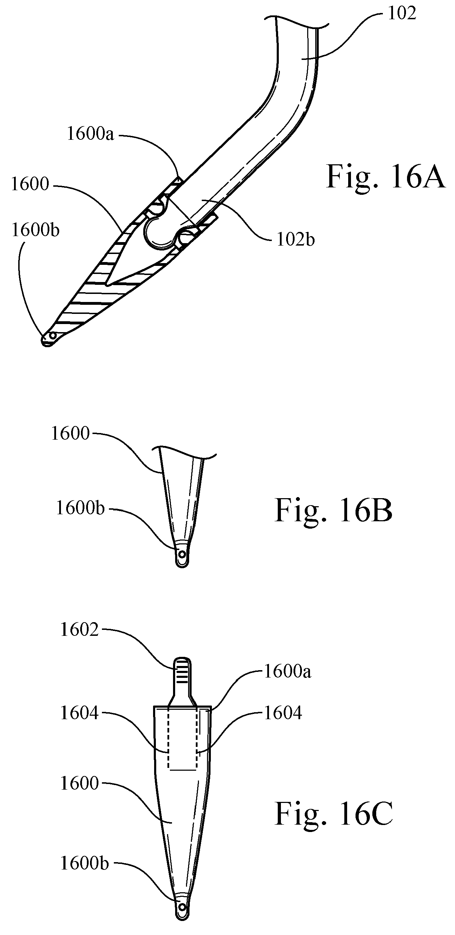

Figs. 16A-16C illustrate another embodiment of a removable tip 1600 for use with retractor 102 and is similar to the embodiment discussed above in connection with Figs. 4A-4D. Specifically, Fig. 16A illustrates a perspective partial cross-sectional view of exemplary retractor 102 attached to exemplary removable tip 1600, Fig. 16B illustrates a perspective view of the distal end portion of the removable tip 1600, and Fig. 16C illustrates a perspective view of removable tip 1600 disengaged from the exemplary retractor.

Referring to Figs. 16A and 16B, removable tip 1600 includes proximal portion 1600a and distal portion 1600b. Removable tip 1600 is a substantially conical-shaped removable piece configured for attachment to distal portion 102b of retractor 102 to improve retractor insertion, especially in the case of patients having large amounts of adipose tissue. As best seen in Fig. 16, distal portion 1600b of removable tip 1600 comprises a flattened end portion having a reduced cross-section for ease of insertion and navigation through an incision and to prevent damaging organs and tissue. The reduced cross-section also facilitates grasping by a forceps or other device. Distal portion 1600b may also include a opening for the attachment of a suture or pull wire.

Referring to Fig. 16A, proximal portion 1600a of removable tip 1600 is substantially hollow and configured for receiving distal portion 102b of retractor 102, via, for example, a snap fit or other attachment means, including but not limited to a friction fit. In the particular embodiment illustrated, the distal portion 102b of the retractor 102 comprises one or more grooves or indentations that are configured to receive one or more ridges or protrusion on the inside of removable tip 1600 to facilitate engagement between therebetween.

As illustrated in Fig. 16C, removable tip 1600 includes pull tab 1602 that when pulled, breaks removable tip 1600 at perforations 1604, thereby causing removable tip 1600 to release its grip on retractor 102 such that removable tip 1600 may be removed. As explained above in connection with the embodiment of Figs. 4C-4D, the pull tab 1602 can be grasped with a forceps or endo gripper, and then pulled away from removable tip 1600 to thereby cause perforations 1604 to break. Removable tip 1600 can then be disengaged from the retractor 102 and removed through an incision or port, or after the procedure is complete. Once removable tip 1600 is disengaged from retractor 102, retractor 102 alone is of a sufficient length to perform its retracting functions.

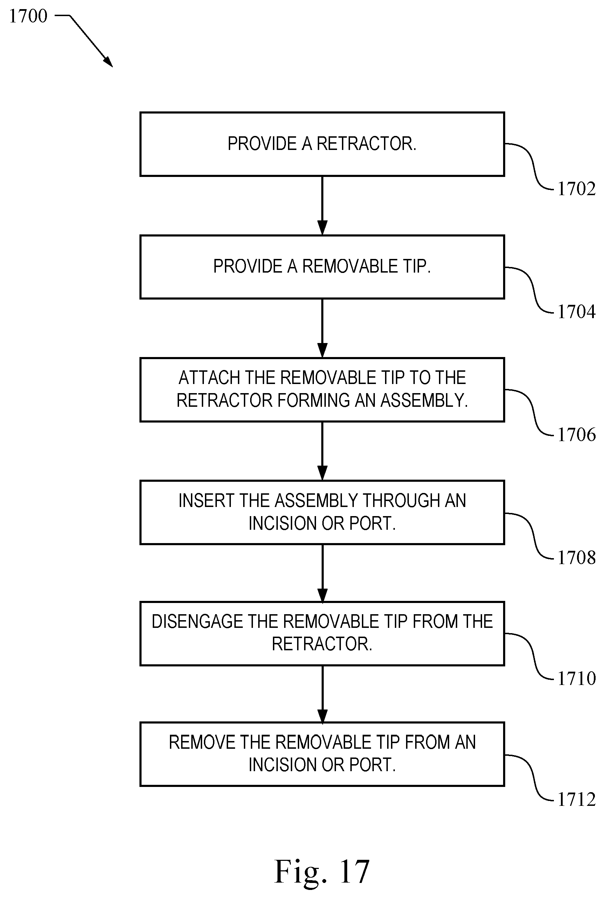

Fig. 17 illustrates a method 1700 of use of an exemplary retractor-removable tip system, such as those illustrated herein and equivalents thereto. At block 1702, a retractor is provided, such as those illustrated in Figs. 1A-16C or equivalents thereto. At block 1704, a removable tip is provided, such as those illustrated in Figs. 1A-16C or equivalents thereto. At block 1706, the removable tip is attached to the retractor forming an assembly. Attachment means include, but are not limited to a snap fit, friction fit, and a screw/threaded attachment. At block 1708 the assembly is inserted through an incision or a port. At block 1710, the removable tip is disengaged from the retractor by a disengagement means, such as those illustrated in Figs. 1A-16C, including but not limited to, pulling the removable tip distally from the retractor, snapping a distal portion of the tip breaking the supporting neck, pulling a pull cord or pull tab causing the removable tip to break at perforations. At block 1712 the removable tip is removed from the incision or port, which may be the same incision or port illustrated at block 1608. The removable tip may be removed before or after removing the retractor from an incision or port. Means for removing the removable tip from an incision or a port include, but are not limited to, those illustrated in Figs. 1A-16C and equivalents thereto, including but not limited to, back-threading the removable tip proximally along the retractor out through the incision or port, removing the retractor through a first incision or port and then removing the removable tip through the first incision or port, and removing the removable tip through a first incision or port and then removing the retractor through a second incision or port.

From the foregoing, the discovery of systems, apparatuses, and methods of removable tips for use in conjunction with medical instruments, including but not limited to, medical retractors in laparoscopic surgery improve the efficiency and navigation to the target anatomy while maintaining adequate exposure to the target anatomy. It can be seen that the embodiments illustrated and equivalents thereto as well as the methods of manufacturer may utilize machines or other resources, such as human beings, thereby reducing the time, labor, and resources required to manufacture the embodiments. Indeed, the discovery is not limited to the embodiments illustrated herein, and the principles and methods illustrated herein may be applied and configured to any retractor and equivalents.

Those of skill in the art will appreciate that embodiments not expressly illustrated herein may be practiced within the scope of the present discovery, including that features illustrated herein for different embodiments may be combined with each other and/or with currently-known or future-developed technologies while remaining within the scope of the claims presented here. It is therefore intended that the foregoing detailed description be regarded as illustrative rather than limiting. It is understood that the following claims, including all equivalents, are intended to define the scope of the discovery. Furthermore, the advantages illustrated above are not necessarily the only advantages of the discovery, and it is not necessarily expected that all of the illustrated advantages will be achieved with every embodiment of the discovery.

## Claims

1. A removable tip comprising:
a substantially conical-shaped body (200, 300, 400, 500, 800, 1500) comprising a proximal body portion and a distal body portion, wherein the proximal body portion comprises a substantially hollow portion and is configured for receiving a distal portion of a medical retractor, and wherein the distal body portion is atraumatic; further comprising disengagement means for disengagement of the removable tip from a medical retractor, **characterised in that** the disengagement means comprises at least one of a snap point (204) to break a supporting neck of the removable tip, a pull cord (302, 502) to break a perforation of the removable tip, or a pull tab (402) to break a perforation of the removable tip.

2. The removable tip of claim 1 further comprising: a supporting neck disposed between the proximal body portion and the distal body portion; wherein the snap point is configured to break a portion of the supporting neck when a force is applied to the distal body portion away from the snap point.

3. The removable tip of claim 1, wherein the pull cord is attached to the substantially conical-shaped body; and one or more perforations are disposed on the substantially conical-shaped body and near to the pull cord configured to break when the pull cord is pulled in a direction proximal to the substantially conical-shaped body.

4. The removable tip of Claim 3, wherein said perforations are disposed spirally about the substantially conical-shaped body.

5. The removable tip of Claim 1, Claim 3 or Claim 4 wherein said pull cord further comprises a retaining ring disposed at a proximal portion of the pull cord.

6. The removable tip of claim 1 wherein the pull tab is attached to the substantially conical-shaped body; and one or more perforations are disposed on the substantially conical-shaped body and adjacent to the pull tab configured to break when the pull tab is pulled in a direction away from the substantially conical-shaped body.

7. A medical retractor system comprising the removable tip of any one of the preceding claims and a medical retractor having a proximal portion and a distal portion, wherein the distal portion is connected to the removable tip.

8. The medical retractor system of Claim 7 comprising a plurality of removable tips in accordance with any one of Claims 1 to 6, each of said plurality of removable tips being of a different size.

## Patentansprüche

1. Entfernbare Spitze, umfassend:
einen im Wesentlichen konisch gestalteten Körper (200, 300, 400, 500, 800, 1500), der einen proximalen Körperabschnitt und einen distalen Körperabschnitt umfasst, wobei der proximale Körperabschnitt einen im Wesentlichen hohlen Abschnitt umfasst und dazu konfiguriert ist, einen distalen Abschnitt einer medizinischen Retraktionsvorrichtung aufzunehmen, und wobei der distale Körperabschnitt atraumatisch ist; ferner umfassend ein Ausrückmittel zum Ausrücken der entfernbaren Spitze aus einer medizischen Retraktionsvorrichtung, **dadurch gekennzeichnet, dass** das Ausrückmittel eine Abbrechstelle (204) zum Brechen eines Stützhalses der entfernbaren Spitze und/oder eine Zugschnur (302, 502) zum Brechen einer Perforation der entfernbaren Spitze und/oder eine Zuglasche (402) zum Brechen einer Perforation der entfernbaren Spitze umfasst.

2. Entfernbare Spitze nach Anspruch 1, ferner umfassend einen zwischen dem proximalen Körperabschnitt und dem distalen Körperabschnitt angeordneten Stützhals, wobei die Abbrechstelle dazu konfiguriert ist, dass ein Abschnitt des Stützhalses daran abbricht, wenn eine Kraft von der Abbrechstelle weg auf den distalen Körperabschnitt ausgeübt wird.

3. Entfernbare Spitze nach Anspruch 1, wobei die Zugschnur an dem im Wesentlichen konisch gestalteten Körper angebracht ist und eine oder mehrere Perforationen an dem im Wesentlichen konisch gestalteten Körper und in der Nähe der Zugschnur angeordnet sind, die zum Brechen konfiguriert sind, wenn in einer proximal zu dem im Wesentlichen konisch gestalteten Körper verlaufenden Richtung an der Zugschnur gezogen wird.

4. Entfernbare Spitze nach Anspruch 3, wobei die Perforationen spiralförmig um den im Wesentlichen konisch gestalteten Körper angeordnet sind.

5. Entfernbare Spitze nach Anspruch 1, Anspruch 3 oder Anspruch 4, wobei die Zugschnur ferner einen Haltering umfasst, der an einem proximalen Abschnitt der Zugschnur angeordnet ist.

6. Entfernbare Spitze nach Anspruch 1, wobei die Zuglasche an dem im Wesentlichen konisch gestalteten Körper angebracht ist und eine oder mehrere Perforationen an dem im Wesentlichen konisch gestalteten Körper und in der Nähe der Zuglasche angeordnet sind, die zum Brechen konfiguriert sind, wenn in einer vom konisch gestalteten Körper weg verlaufenden Richtung an der Zuglasche gezogen wird.

7. Medizinisches Retraktionsvorrichtungssystem, umfassend die entfernbare Spitze nach einem der vorhergehenden Ansprüche und eine medizinische Retraktionsvorrichtung mit einem proximalen Abschnitt und einem distalen Abschnitt, wobei der distale Abschnitt mit der entfernbaren Spitze verbunden ist.

8. Medizinisches Retraktionsvorrichtungssystem nach Anspruch 7, umfassend eine Vielzahl von entfernbaren Spitzen nach einem der Ansprüche 1 bis 6, wobei jede der Vielzahl von entfernbaren Spitzen eine andere Größe hat.

## Revendications

1. Pointe amovible comprenant :
un corps essentiellement conique (200, 300, 400, 500, 800, 1500) comprenant une partie de corps proximale et une partie de corps distale, la partie de corps proximale comprenant une partie essentiellement creuse et étant configurée pour recevoir une partie distale d'un écarteur médical, et la partie de corps distale étant atraumatique ; comprenant en outre des moyens de désaccouplement permettant de désaccoupler la pointe amovible d'un écarteur médical, **caractérisée en ce que** les moyens de désaccouplement comprennent au moins un élément parmi un point de rupture (204) pour la rupture d'un col de support de la pointe amovible, un cordon de traction (302, 502) pour la rupture d'une perforation de la pointe amovible, et une languette de traction (402) pour la rupture d'une perforation de la pointe amovible.

2. Pointe amovible selon la revendication 1, comprenant en outre : un col de support disposé entre la partie de corps proximale et la partie de corps distale ; dans laquelle le point de rupture est configuré pour rompre une partie du col de support lorsqu'une force est appliquée à la partie de corps distale à distance du point de rupture.

3. Pointe amovible selon la revendication 1, dans laquelle le cordon de traction est attaché au corps essentiellement conique ; et une ou plusieurs perforations sont disposées sur le corps essentiellement conique et à proximité du cordon de traction, configurées pour rompre lorsque le cordon de traction est tiré dans une direction proximale vis-à-vis du corps essentiellement conique.

4. Pointe amovible selon la revendication 3, dans laquelle lesdites perforations sont disposées en spirale autour du corps essentiellement conique.

5. Pointe amovible selon la revendication 1, la revendication 3 ou la revendication 4, dans laquelle ledit cordon de traction comprend en outre un anneau de retenue disposé au niveau d'une partie proximale du cordon de traction.

6. Pointe amovible selon la revendication 1, dans laquelle la languette de traction est attachée au corps essentiellement conique ; et une ou plusieurs perforations sont disposées sur le corps essentiellement conique et en position adjacente à la languette de traction, configurées pour rompre lorsque la languette de traction est tirée dans une direction s'éloignant du corps essentiellement conique.

7. Système d'écarteur médical comprenant la pointe amovible selon l'une quelconque des revendications précédentes et un écarteur médical comportant une partie proximale et une partie distale, la partie distale étant raccordée à la pointe amovible.

8. Système d'écarteur médical selon la revendication 7, comprenant une pluralité de pointes amovibles selon l'une quelconque des revendications 1 à 6, chaque pointe parmi ladite pluralité de pointes amovibles étant d'une taille différente.
